# EUROPEAN PATENT APPLICATION

(11) **EP 3 563 927 A1**
(43) Date of publication of application: **06.11.2019**
(21) Application number: 18075010.1
(22) Date of filing: 17.07.2018
(51) Int. Cl.: B01D 61/02, C07D 301/32, C08G 59/02, C08G 59/32, C08L 63/00

(54) **PURIFICATION OF HIGH PERFORMANCE EPOXY RESINS VIA MEMBRANE FILTRATION TECHNOLOGY**

(30) Priority: 30.04.2018 EP 18075005
(71) Applicant: Hexion Research Belgium SA, 1348 Ottignies-Louvain-la-Neuve (BE)
(72) Inventor: The designation of the inventor has not yet been filed

(57) **Abstract**

The invention describes a low temperature process for high performance epoxy resins purification via membrane separation technology. Continuous or semi-continuous low temperature processing grants a minimized material aging during product purification as for example glycidyl amine based resins.

## Description

### FIELD OF THE DISCLOSURE

The invention describes a low temperature process for high performance epoxy resins purification via membrane separation technology. Continuous or semi-continuous low temperature processing grants a minimized material aging as well as a safe handling of high reactive chemicals and its thermal instable side streams, that are accumulated during product purification as for example glycidyl amine based resins.

### BACKGROUND OF THE DISCLOSURE

Current status quo in industrial epoxy resin manufacture is still with focus on common batch/semi-batch technology. The technology in use limits the product selectivity towards the desired monomer content, caused by unwanted side reactions and related formation of side products as a consequence. In order to generate high performance in thermoset resins for e.g. aerospace application, glycidyl amine based epoxy resins need further purification, subsequently to the manufacturing process to increase the monomer content. This purification is performed by high temperature molecular distillation processing. In a two-staged thin-film evaporation unit the crude epoxy resin is first separated from its light ends ("topping process") and thereafter at even higher temperatures separated from its heavy end products ("tailing process"). From a commercial perspective, the physical distillation process is costly in terms of high energy consumption (processing temperatures of 140-220 °C are required) as well as a high safety system / safe guarding standards requirements (prevention of thermal runaway in high temperature processing with thermally instable epoxy resins). Furthermore, the high temperature processing leads to product aging / degradation and as a result to yield losses. On addition the handling of purification side streams is dangerous and costly.

### SUMMARY OF THE DISCLOSURE

The invention describes a low temperature process concept for glycidyl amine based high performance epoxy resins purification via membrane separation technology. Continuous or semi-continuous low temperature processing grants a minimized material aging as well as a safe handling of the high reactive chemicals.

### BRIEF DESCRIPTION OF THE DRAWINGS

For a further understanding of the nature and objects of the present invention, reference may be had to the following detailed description taken in conjunction with the accompanying figure, wherein:
Figure 1: Schematic flow chart of production concepts
Figure 2: Test results to proof the purification of glycidyl amines via membrane technology. HPLC graphs of EP 499 and membrane separated permeate streams of an NF membrane of choice at different pressures are compared.
Figure 3: HPLC finger print comparison of competitor technologies in use. Common physical molecular distillation vs. membrane filtration.

### DETAILED DESCRIPTION OF THE DISCLOSURE

High-performance fibers combined with glycidyl amine based thermoset resins offer very high strength-to-weight ratios and are ideal for manufacturing of lightweight storage vessels, pressure vessels and / or other composite structures and articles. Structural aerospace components are one of the most critical and demanding applications with regards to quality in terms of precision and tolerances.

This invention claims a continuous or semi-continuous, low temperature purification process for high performance resins, thermoset resins or base epoxy resins in general and more specifically glycidyl amine based high performance resins.

The process combines
- a single membrane filtration unit that operates at low solvent concentrations (5% max) which could be required to reduce resin viscosities and to "wash out" the purified resin through a common purchasable membrane of choice -
- or cascaded membrane filtration units that operate high solvent concentrations (up to 70% max) for optimized flux/yield rates
combine at the end of the process with current physical distillation units such as flashers or wiped / thin film evaporator devices (TFE) to remove the solvents of choice from the purified resin, thereafter.

The membrane filtration units of choice purify manufactured crude epoxy resins from unwanted light-, heavy-end side products and used process solvents, to yield a wanted monomer concentration of approx. 88-95%.

Within the membrane purification process the feed-stream consists of an epoxy resin to be purified and a solvent of choice (e.g. benzylic alcohol, methylisobutyl ketone, acetone, toluene), low viscous epoxy resins or epoxy functional diluents.

The membrane does separate/purify/split the crude epoxy resin feed-stream into a purified resin stream - named permeate and a back-cycled stream - named retentate.

The permeate stream consists of purified epoxy resin (88-95% wanted monomer) and its corresponding solvent of choice (up to 5% in case of single unit operation; up to 70% in case of cascade operation).

The back-cycled/looped retentate stream consists of crude residual epoxy resin to be further purified, accumulated residue and its corresponding solvent of choice and/or low viscous epoxy resins or epoxy functional diluents.

The membrane units of choice consist of either common purchasable flat sheets, polymeric membranes, which are coiled - forming a tubular battery unit or consist of tubular ceramic membranes.

The separation process is performed under moderate operating pressures of 10-40 bar and low/moderate process temperatures of 23-80°C. To remove unwanted residual solvents from the purified epoxy resin, either a flasher unit or a TFE device is integrated into the manufacturing process, subsequently to the membrane filtration unit of choice. The physical distillation units (flasher, TFE) operate in a temperature range of 100-160°C and pressures of approx. 0.2-0.01 bar minimum.

The retentate streams (unwanted side products; solvents) separated via the membrane filtration units from the purified epoxy resin get back-cycled to allow continuous/semi-continuous processing.

Low temperature, continuous or semi-continuous purification benefits by lowering production costs (less energy consumption), increased product performance related to lower thermal stress during processing as well as increased process safety (low/no thermal runaway risk of thermal instable residue streams).

By introducing a low viscous epoxy resin or epoxy functional diluent into the crude epoxy resin feed-stream prior to the membrane filtration operation, a unique new epoxy resin compound or side stream of improved performance (e.g. higher Tg) for composite application could be obtained. The low viscous epoxy resin of choice is constituted in its molecular shape in that way, that it does not permeate through the membrane but is retained in the retentate stream with diluting the obtained high chemical reactive residues and increases its thermal stability.

The low viscous epoxy resin or epoxy functional diluent retained in the retentate stream increases the thermal stability of the high chemical reactive residue.

### EXAMPLES

The following examples and comparative examples are provided to illustrate certain embodiments of the invention.

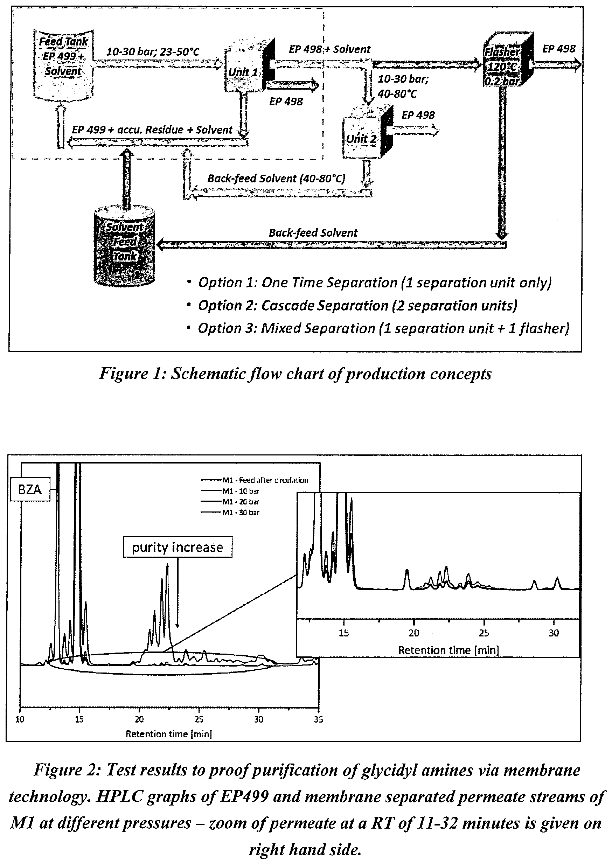

Figure 1 describes different possible future manufacturing options using membrane filtration technology to purify glycidyl amines and / or other epoxy resins.
Option 1 (blue boxed) operates with just one filtration unit and low solvent concentrations up to 5% max. The permeate contains the purified glycidyl amine EP 498 and solvent. The retentate contains crude EP499 glycidyl amine, separated residue and solvent is back-cycled into the feeding tank, that feeds the membrane filtration unit. The low solvent concentration (approx. 5% at maximum) does allow to introduce a flasher (option 3 - orange) subsequently after the filtration unit to further purify the EP498 from remaining solvent. The solvent can also be back-feeded into a solvent tank and the retentate stream, respectively.
Option 2 (green) describes a cascaded filtration operation that requires high amounts of solvent (up to 70%) but works with an optimized flux rate/yield caused by the increased solvent amount i.e. compared to option 1. However, the high amount of solvent obtained after first membrane filtration unit does not allow the connection to a flasher for solvent removal. Furthermore, a second filtration unit needs to be cascaded in which the purified glycidyl amine EP498 stream is separated from the solvent. The solvent can be back-feeded into the retentate stream of the first membrane filtration unit. In option 2, a flasher can be introduced (if needed) as well subsequent to the second filtration unit to remove residual solvent as well.

### MEMBRANES

Four commercial NF membranes (A4 size) with nominal molecular weight cut-offs (MWCO) matching with the molar masses of the product compound and the impurities were selected: three flat sheet polymeric OSN membranes recommended for use in non-polar solvents, i.e. PuraMem Performance (Evonik), PuraMem 280 (Evonik) and NF010206 (Solsep), and one tubular ceramic membrane, i.e. 0.9 nm TiO₂ (Inopor).

All three polymeric membranes were tested as flat sheets (rectangular coupons of approx. 100 cm²), however they are equally available as spiral-wound elements with various dimensions and surface areas. The ceramic membrane was a 1-channel tube with active titanium top layer at the lumen side, outer diameter of 10 mm, inner diameter of 7 mm and length of 12 cm, providing a surface area of approx. 25 cm².

### TEST PROTOCOL

At the onset of the experimental campaign, the internal circuit of the test rig and the membrane housings were thoroughly rinsed with acetone and subsequently blow-dried using nitrogen gas.

The selected membrane was installed in its housing which was then mounted in the filtration rig using quick connectors. Prior to the actual screening trials on (solvent based) resin test mixtures, the polymeric membranes were preconditioned by permeation of at least 50 ml of pure benzyl alcohol (according to instructions of membrane supplier) to wash out the preservatives used for dry storage, after which the membrane coupon kept wet. The ceramic membrane was used without pretreatment.

Approx. 3-4 L of test liquid was applied into the feed tank and circulated at approx. 23°C (trials on benzyl alcohol based resin mixtures) or 40°C (trials on solvent-free sample, approx. 5 h). After temperature stabilization, a Feed sample (approx. 5 ml) was taken and the test mixture was pressurized using nitrogen gas. In case of the 1 wt.% resin test mixture, each membrane was consecutively tested at three (trans membrane pressure) TMPs, i.e. 10 bar, 20 bar and 30 bar, maintaining the feed flow at approx. 600 I.h-1. Permeate/Retentate samples (approx. 5 mL) were taken at steady-state conditions (stable flux) for all conditions studied, except for the trials on concentrated feed mixtures where fluxes were so low that only mix Permeate samples could be taken (collection of permeate from first droplet onward). After sampling, the remainder of the permeate was reintroduced in the feed tank to reconstitute the feed. As the trials were conducted in regular filtration mode at low permeate recovery, quasi iso-concentration conditions at the feed side can be assumed. Permeate fluxes, TMPs and temperatures were monitored and recorded in real time.

PuraMem Performance was tested on various resin mixtures and a fresh preconditioned membrane coupon was used for each test mixture.

All permeate samples collected were visually colorless, except for those of NF0101206 which were slightly yellowish.

All samples were analyzed by HPLC. The concentration of the desired monomer resin compound in the permeate samples of PuraMem Performance membrane is in the range 87-92%, which is significantly higher than the product content in the crude resin (approx. 77%) and close to the purity target (92%). The chromatograms show that especially the higher Mw impurities are removed to a high extent, with retentions decreasing slightly with increasing TMP. Also the permeates of the other membranes tested were significantly enriched in the target resin product, however not to the same extent as those of PuraMem Performance. No visual signs of membrane fouling were observed after the trials.

Figure 2 describes test results to proof the purification of glycidyl amines via membrane technology. HPLC graphs of EP 499 and membrane separated permeate streams of an NF membrane of choice at different pressures are compared.

Via HPLC analytics it has been proven, that unpurified crude glycidyl amine EP 499 (black) could be highly purified (up to 88-92%) with membrane filtration at different pressures (red - 10 bar, blue - 20 bar, pink - 30 bar) to yield the wanted EP 498 epoxy resin.

At a retention time (RT) of about 13 minutes the solvent of choice (benzylic alcohol in this case) is detected. The purified glycidyl amine EP 498 eluates at a RT of 15 minutes. Oligomeric impurities that eluate at a RT of 20-30 minutes are significantly reduced via membrane filtration. Operating the filtration at different pressures does impact/improve the flux rate/yield but not the selectivity of the filtration operation.

To proof the innovation newness and uniqueness of low temperature membrane filtration, several competitor samples have been compared with Hexion manufactured EP 498 using high temperature molecular distillation technology vs. material that has been purified via membrane technology.

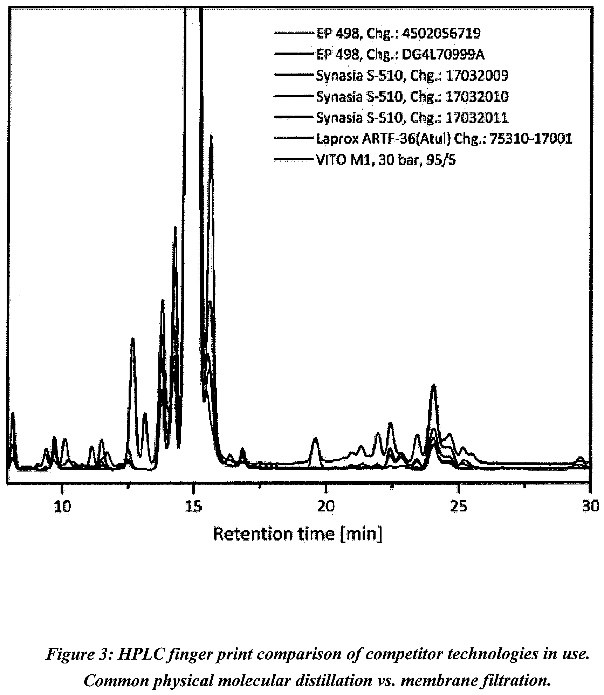

Figure 3 shows HPLC traces of purified glycidyl amine EP 498 using different purification technologies. The purple chromatograph reflects material that got purified using membrane technology. It clearly shows a different fingerprint trace if compared to all other traces shown.

The black and the red chromatograph are obtained for purified glycidyl amine EP 498 manufactured by Hexion that has been purified using physical molecular thin film distillation technology. The red/black graphs do overlay/match the traces of tested competitors materials like e.g. Synasia (who did copy Huntsman technology) shown in blue, pink and green as well as Atul reflected in dark blue. Thus does proof two points: a) membrane filtration technology purified resin shows a unique HPLC fingerprint and b) all compared competitors do currently use common physical distillation technology with the disadvantage of treating the epoxy Resin with much higher thermal stress compared to low temperature membrane filtration technology.

Specifically the light end impurity level of competitor material has been detected much higher in comparison to material purified via membrane filtration technology (RT 8-12 min).

## Claims

1. A continuous or semi-continuous, low temperature purification process for high performance resins, thermoset resins or epoxy resins and more specifically glycidyl amine based high performance resins,
the process combines :
- a single membrane filtration unit that operates at low solvent concentrations
- or cascaded membrane filtration units that operate high to medium solvent concentrations
combine at the end of the process with current physical distillation units such as flashers or wiped thin film evaporator devices to remove the solvents of the purified resin, thereafter.

2. The process of claim 1, wherein the membrane or a cascade of membranes is **characterized in** a polymeric OSN membrane or tubular ceramic membrane, and combinations thereof.

3. The process of claim 1, wherein the solvent used in a concentration from 5 wt% to up to 70 wt%.

4. The process of claim 1, wherein the solvent used is compatible with the membranes in a concentration from 5 wt% to up to 70 wt%.

5. The process of claim 1, wherein the solvent used in a concentration from 5 wt% to up to 70 wt% is an alcohol, a ketone, an aromatic solvent, a low viscous epoxy resin or epoxy functional diluents or a combination thereof.

6. The process of claim 1, wherein the solvent is selected from benzyl alcohol, acetone, methyl isbutyl ketone, toluene, xylene or a combination thereof.

7. The process of claim 1, wherein the temperature applied to the membrane or a cascade of membranes is between room temperature to 70°C.

8. A high performance resin, thermoset resin or epoxy resin and more specifically glycidyl amine based high performance resin, purified by the process of one of preceding claims.

9. The thermosetting composition based on the resins of claim 8 used in combination with fibrous material to produce a high performance composite matrix.

10. The process of claim 1, wherein an epoxy resin or diluent is added to the feed-stream, which is constituted as such that it does not permeate through the membrane but forms a new product in the retentate stream with unique features (e.g. increased Tg) and increased thermal stability compared to the residue stream formed in common physical molecular distillation operations.
